# EUROPEAN PATENT APPLICATION

(11) **EP 3 967 707 A1**
(43) Date of publication of application: **16.03.2022**
(21) Application number: 20177319.9
(22) Date of filing: 30.10.2016
(51) Int. Cl.: C07K 16/00, C07K 14/62, A61K 38/17, A61K 38/18, A61K 38/19, A61K 38/22

(54) **A METHOD FOR EXTENDING HALF-LIFE OF A PROTEIN**

(30) Priority: 16.11.2015 KR 20150160728
(62) Divisional of application: 16866579.2
(71) Applicant: Ubiprotein, Corp., Seongnam-si, Gyeonggi-do 13493 (KR)
(72) Inventor: KIM, Kyunggon, Seoul 05507 (KR); BAEK, Kwang-Hyun, Seoul 06291 (KR); BAE, Sung-ryul, Seongnam-si Gyeonggi-do 13602 (KR); KIM, Myung-Sun, Wonju-si Gangwon-do 26438 (KR); KIM, Hyeonmi, Suwon-si Gyeonggi-do 16687 (KR); YOO, Yeeun, Guri-si Gyeonggi-do 11940 (KR); LI, Lan, Tangshan, Hebei, 063000 (CN); PARK, Jung-Hyun, Seongnam-si, Gyeonggi-do 13493 (KR); KIM, Jin-Ok, Jeungpyeong-gun Chungcheongbuk-do 27912 (KR)
(74) Representative: Bringer IP

(57) **Abstract**

The present invention relates to a method for prolonging half-life of a protein or a (poly)peptide by replacing one or more lysine residues of the protein related to ubiquitination, and the protein having a prolonged half-life.

## Description

### Technical Field

The present invention relates to a method for prolonging half-life of a protein or a (poly)peptide by replacing one or more lysine residues of the protein related to ubiquitination, and the protein having a prolonged half-life.

### Background Art

A protein or (poly)peptide in eukaryotic cells is degraded through two distinct pathways of lysosomal system and ubiquitin-proteasome system. The lysosomal system, in which 10 to 20% cellular proteins are decomposed, has neither substrate specificity nor precise timing controllability. That is, the lysosomal system is a process to break down especially most of extracellular proteins or membrane proteins, as surface proteins are engulfed by endocytosis and degraded by the lysosome. For the selective degradation of a protein in eukaryotic cells, ubiquitin-proteasome pathway (UPP) should be involved, wherein the target protein is first bound to ubiquitin-binding enzyme to form poly-ubiquitin chain, and then recognized and decomposed by proteasome. About 80 to 90% of eukaryotic cell proteins are degraded through UPP, and thus it is considered that the UPP regulates degradation for most of cellular proteins in eukaryotes, and presides over protein turnover and homeostasis in vivo. The ubiquitin is a small protein consisting of highly conserved 76 amino acids and it exists in all eukaryotic cells. Among the amino acid residues of the ubiquitin, the residues at positions corresponding to 6, 11, 27, 29, 33, 48 and 63 are lysines (Lysine, Lys, K), and the residues at positions 48 and 63 are known to have essential roles in the formation of poly-ubiquitin chain. The three enzymes, known generically as E1, E2 and E3, act in series to promote ubiquitination, and the ubiquitin-tagged proteins are decomposed by the 26S proteasome of ATP-dependent protein degradation complex.

As disclosed above, the ubiquitinproteasome pathway (UPP) consists of two discrete and continuous processes. One is protein tagging process in which a number of ubiquitin molecules are conjugated to the substrate proteins, and the other is degradation process where the tagged proteins are broken down by the 26S proteasome complex. The conjugation between the ubiquitin and the substrate protein is implemented by the formation of isopeptide bond between C-terminus glycine of the ubiquitin and lysine residue of the substrate, and followed by thiol-ester bond development between the ubiquitin and the substrate protein by a series of enzymes of ubiquitin-activating enzyme E1, ubiquitin-binding enzyme E2 and ubiquitin ligase E3. The E1 (ubiquitin-activating enzyme) is known to activate ubiquitin through ATP-dependent reaction mechanism. The activated ubiquitin is transferred to cysteine residue in the ubiquitin-conjugation domain of the E2 (ubiquitin-conjugating enzyme), and then the E2 delivers the activated ubiquitin to E3 ligase or to the substrate protein directly. The E3 also catalyzes stable isopeptide bond formation between lysine residue of the substrate protein and glycine of the ubiquitin. Another ubquitin can be conjugated to the C-terminus lysine residue of the ubiquitin bound to the substrate protein, and the repetitive conjugation of additional ubiquitin moieties as such produces a poly-ubiquitin chain in which a number of ubiquitin molecules are linked to one another. If the poly-ubquitin chain is produced, then the substrate protein is selectively recognized and degraded by the 26S proteasome.

Meanwhile, there are various kinds of proteins which have therapeutic effects in vivo. The proteins or (poly)peptides or bioactive polypeptides having therapeutic effects in vivo include, but not limited, for example, growth hormone releasing hormone (GHRH), growth hormone releasing peptide, interferons (interferon-a or interferon-β), interferon receptors, colony stimulating factors (CSFs), glucagon-like peptides, interleukins, interleukin receptors, enzymes, interleukin binding proteins, cytokine binding proteins, G-protein-coupled receptor, human growth hormone (hGH), macrophage activating factor, macrophage peptide, B cell factor, T cell factor, protein A, allergy inhibitor, cell necrosis glycoproteins, G-protein-coupled receptor, immunotoxin, lymphotoxin, tumor necrosis factor, tumor suppressors, metastasis growth factor, alpha-1 antitrypsin, albumin, alpha-lactalbumin, apolipoprotein-E, erythropoietin, highly glycosylated erythropoietin, angiopoietins, hemoglobin, thrombin, thrombin receptor activating peptide, thrombomodulin, factor VII, factor VIIa, factor VIII, factor IX, factor XIII, plasminogen activating factor, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone growth factor, bone stimulating protein, calcitonin, insulin, atriopeptin, cartilage inducing factor, fibrin-binding peptide, elcatonin, connective tissue activating factor, tissue factor pathway inhibitor, follicle stimulating hormone, luteinizing hormone, luteinizing hormone releasing hormone, nerve growth factors, parathyroid hormone, relaxin, secretin, somatomedin, insulin-like growth factor, adrenocortical hormone, glucagon, cholecystokinin, pancreatic polypeptide, gastrin releasing peptide, corticotropin releasing factor, thyroid stimulating hormone, autotaxin, lactoferrin, myostatin, receptors, receptor antagonists, cell surface antigens, virus derived vaccine antigens, monoclonal antibodies, polyclonal antibodies, and antibody fragments. Immunoglobulin G (IgG) is a type of antibody and it is the main type of antibody found in blood and extracellular fluid allowing it to control infection of body tissues, and is secreted as a monomer that is small in size allowing it to easily perfuse tissues (Basic Histology, McGraw-Hill, ISBN 0-8385-0590-2, 2003). IgG is used to treat immune deficiencies, autoimmune disorders, and infections (Proc Natl Acad Sci U S A., 107(46), 19985-19990, 2010).

The protein therapeutic agents relating to homeostasis in vivo have various adverse effects, such as increasing the risk for cancer inducement. For example, possible inducement of thyroid cancer was raised for the incretin degrading enzyme (DPP-4) (Dipeptidyl peptidase-4) inhibitors family therapeutic agents, and insulin glargine was known to increase the breast cancer risk. Further, it was reported that continuous or excessive administration of the growth hormone into the patients suffering from a disease of growth hormone secretion disorder is involved in diabetes, microvascular disorders and premature death of the patients. In this regard, there have been broad studies to reduce such adverse and side effects of the therapeutic proteins. To prolong half-life of the proteins was suggested as a method to minimize the risk of the adverse and side effects of the therapeutic proteins. For this purpose, various methods have been disclosed. In this regard, we, inventors have studied to develop a novel method for prolonging half-life of the proteins in vivo and/or in vitro and completed the present invention by replacing one or more lysine residues related to ubiquitination of the therapeutic proteins or (poly)peptide to prevent the proteins or (poly)peptide degradation through ubiquitine-proteasome system.

The teachings of all patents, published applications and references cited herein are incorporated by reference in their entirety.

### Disclosure of Invention

### Technical Problem

The purpose of the present invention is to enhance half-life of the proteins or (poly)peptide.

Further, another purpose of the present invention is to provide a therapeutic protein having prolonged half-life.

Further, another purpose of the present invention is to provide a pharmaceutical composition comprising the protein having prolonged half-life as a pharmacological active ingredient. Solution to Problem

In order to achieve the purpose, this invention provides a method for extending protein half-life in vivo and/or in vitro by replacing one or more lysine residues on the amino acids of the protein.

In the present invention, the lysine residue can be replaced by conservative amino acid. The term "conservative amino acid replacement" means that an amino acid is replaced by another amino acid which is different from the amino acid to be replaced but has similar chemical features, such as charge or hydrophobic property. The functional features of a protein are not essentially changed by the amino acid replacement using the corresponding conservative amino acid, in general. For example, amino acids can be classified according to the side chains having similar chemical properties, as follows: ① aliphatic side chain: Glycine, Alanine, Valine, Leucine, and Isoleucine; ② aliphatic-hydroxyl side chain: Serine and Threonine; ③ Amide containing side chain: Asparagine and Glutamine; ④ aromatic side chain: Phenyl alanine, Tyrosine, Tryptophan; ⑤ basic side chain: Lysine, Arginine and Histidine; ⑥ Acidic side chain; Aspartate and Glutamate; and ⑦ sulfur-containing side chain: Cysteine and Methionine.

In the present invention, the lysine residue can be substituted with arginine or histidine which contains basic side chain. Preferably, the lysine residue is replaced by arginine.

### Advantageous Effects of Invention

In accordance with the present invention, the mutated protein of which one or more lysine residues are substituted with arginine has significantly prolonged half-life, and thus can remain for a long time.

### Brief Description of Drawings

Figure 99 shows the structure of IgG heavy chain expression vector.

Figure 100 represents the results of cloning for the IgG heavy chain gene.

Figure 101 shows the expression of IgG heavy chain plasmid genes in the HEK-293T cells.

Figure 102 explains the proteolytic pathway of the IgG heavy chain via ubiquitination assay.

Figure 103 shows the ubiquitination levels of the substituted IgG heavy chain of which lysine residue(s) is replace by arginine(s), in comparison to the wild type.

Figure 104 shows the IgG heavy chain half-life change after the treatment with protein synthesis inhibitor cyclohexamide (CHX).

Figure 105 shows the structure of IgG light chain expression vector.

Figure 106 represents the results of cloning for the IgG light chain gene.

Figure 107 shows the expression of IgG light chain plasmid genes in the HEK-293T cells.

Figure 108 explains the proteolytic pathway of the IgG light chain via ubiquitination assay.

Figure 109 shows the ubiquitination levels of the substituted IgG light chain of which lysine residue(s) is replace by arginine(s), in comparison to the wild type.

Figure 110 shows the IgG light chain half-life change after the treatment with protein synthesis inhibitor cyclohexamide (CHX).

Hereinafter, the present invention will be described in more detail with reference to Examples. It should be understood that these examples are not to be in any way construed as limiting the present invention.

In yet another embodiment of the present invention, the protein is IgG. In the amino acid sequence (SEQ No. 97) of the IgG heavy chain, at least one lysine residues at positions corresponding to 49, 62, 84, 95, 143, 155, 169, 227, 232, 235, 236, 240, 244, 268, 270, 296, 310, 312, 339, 342, 344, 348, 356, 360, 362, 382, 392, 414, 431, 436 and 461 from the N-terminus are replaced by arginine. As a result, the IgG having enhanced half-life and the pharmaceutical composition comprising thereof are provided to prevent and/or treat cancer.

In yet another embodiment of the present invention, the protein is IgG. In the amino acid sequence (SEQ No. 104) of the IgG light chain, at least one lysine residues at positions corresponding to 61, 64, 67, 125, 129, 148, 167, 171, 191, 205, 210, 212 and 229 from the N-terminus are replaced by arginine. As a result, the IgG having enhanced half-life and the pharmaceutical composition comprising thereof are provided to prevent and/or treat cancer.

In the present invention, site-directed mutagenesis is employed to substitute lysine residue with arginine (R) residue of the amino acid sequence of the protein. According to this method, primer sets are prepared using DNA sequences to induce site-directed mutagenesis, and then PCR is performed under the certain conditions to produce mutant plasmid DNAs.

In the present invention, the degree of ubiquitination was determined by transfecting a cell line with the target protein by using immunoprecipitation. If the ubiquitination level increases in the transfected cell line after MG132 reagent treatment, it is understood that the target protein is degraded through ubiquitin-proteasome pathway.

The pharmaceutical composition of the president is invention can be administered into a body through various ways including oral, transcutaneous, subcutaneous, intravenous, or intramuscular administration, and more preferably can be administered as an injection type preparation. Further, the pharmaceutical composition of the present invention can be formulated using the method well known to the skilled in the art to provide rapid, sustained or delayed release of the active ingredient following the administration thereof. The formulations may be in the form of a tablet, pill, powder, sachet, elixir, suspension, emulsion, solution, syrup, aerosol, soft and hard gelatin capsule, sterile injectable solution, sterile packaged powder and the like. Examples of suitable carriers, excipients, and diluents are lactose, dextrose, sucrose, mannitol, xylitol, erythritol, maltitol, starches, gum acacia, alginates, gelatin, calcium phosphate, calcium silicate, cellulose, methyl cellulose, microcrystalline cellulose, polyvinyl pyrrolidone, water, methylhydroxybenzoates, propylhydroxybenzoates, talc, magnesium stearate and mineral oil. Further, the formulations may additionally include fillers, anti-agglutinating agents, lubricating agents, wetting agents, favoring agents, emulsifiers, preservatives and the like.

Examples of suitable carriers, excipients, and diluents are lactose, dextrose, sucrose, mannitol, xylitol, erythritol, maltitol, starches, gum acacia, alginates, gelatin, calcium phosphate, calcium silicate, cellulose, methyl cellulose, microcrystalline cellulose, polyvinyl pyrrolidone, water, methylhydroxybenzoates, propylhydroxybenzoates, talc, magnesium stearate and mineral oil. Further, the formulations may additionally include fillers, anti-agglutinating agents, lubricating agents, wetting agents, favoring agents, emulsifiers, preservatives and the like.

As used herein, the singular forms "a," "an," and "the" are intended to include the plural forms as well, unless the context clearly indicates otherwise. Furthermore, to the extent that the terms "including," "includes," "having," "has," "with," "such as," or variants thereof, are used in either the specification and/or the claims, such terms are not limiting and are intended to be inclusive in a manner similar to the term "comprising". In the present invention, the "bioactive polypeptide or protein" is the (poly)peptide or protein representing useful biological activity when it is administered into a mammal including human.

### Mode for the Invention

The following examples provide illustrative embodiments. In light of the present disclosure and the general level of skill in the art, those of skill will appreciate that the following examples are intended to be exemplary only and that numerous changes, modifcations, and alterations can be employed without departing from the scope of the presently claimed subject matter.

### Example 15: The analysis of ubiquitination and half-life increase of IgG heavy chain, and the analysis of signal transduction in cells.

### 1. IgG heavy chain expression vector cloning and protein expression

### (1) IgG heavy chain expression vector cloning

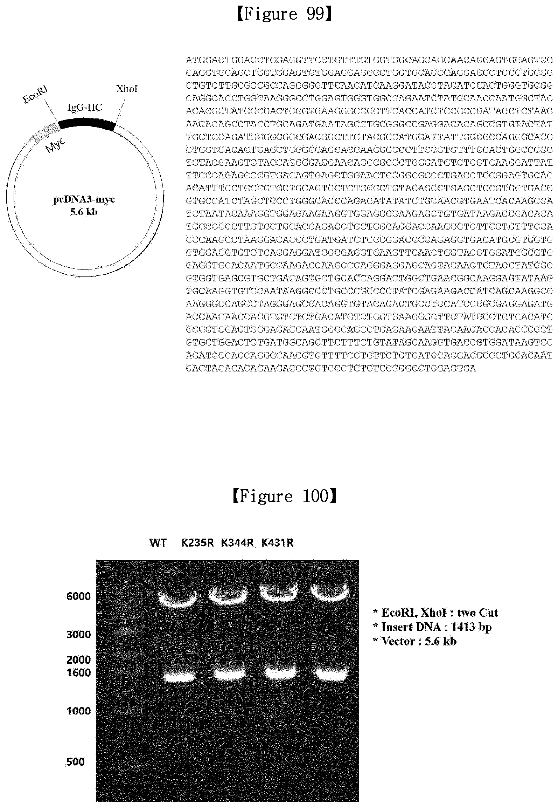

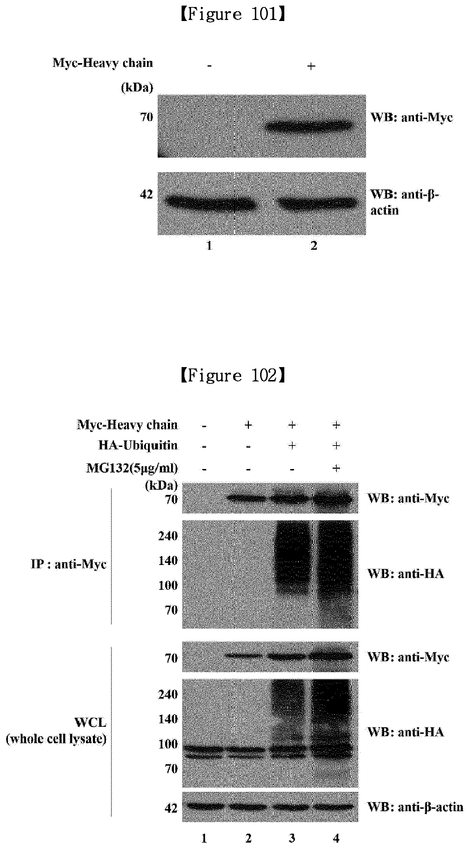

The IgG heavy chain (HC) DNA sequence was synthesized in accordance with the description of Roche's EP1308455 B9 (A composition comprising anti-HER2 antibodies, p. 24), and further optimized to express well in a mammalian cell. Then, IgG heavy chain (HC) DNA amplified by PCR was treated with EcoRI and XhoI, and then ligated to pcDNA3-myc vector (5.6 kb) previously digested with the same enzyme (Fig. 99, IgG heavy chain amnio acid sequence: SEQ No. 97). Then, agarose gel electrophoresis was carried out to confirm the presence of the DNA insert, after restriction enzyme digestion of the cloned vector (Fig. 100). For the assessment of the expression of proteins encoded by cloned DNA, western blot was carried out with anti-myc antibody (9E10, sc-40) to myc of pcDNA3-myc vector shown in the map of Fig. 99. The western blot result showed that the IgG heavy chain (HC) bound to myc was expressed well. The normalization with actin assured that proper amount of protein was loaded (Fig. 101).

### (2) Lysine (Lysine, K) residue substitution

Lysine residue was replaced with arginine (Arginine, R) using site-directed mutagenesis. The following primer sets were used for PCR to prepare the substituted plasmid DNAs.

(IgG HC K235R) FP 5'-ACAAAGGTGGACAGGAAGGTGGAGCCCAAG-3' (SEQ No. 98), RP 5'-CTTGGGCTCCACCTTCC TGTCCACCTTTGT-3' (SEQ No. 99);

(IgG HC K344R) FP 5'- GAGTATAAGTGCAGGGTGTCCAATAAGGCCCTGC-3' (SEQ No. 100), RP 5'-GCAGGGCCTTATTGGACACCCTGCACTTATACTC-3' (SEQ No. 101); and

(IgG HC K431R) FP 5'-CTTTCTGTATAGCAGGCTGA CCGTGGATAAGTCC-3' (SEQ No. 102), RP 5'-GGACTTATCCACGGTCAGCCTGCTATACAGAAAG-3' (SEQ No. 103)

Three plasmid DNAs each of which one or more lysine residues were replaced with arginine (K→R) were prepared by using pcDNA3-myc-IgG HC DNA as a template (Table 14).

**[Table 14]**

| Lysine(K) residue site | IgG HC construct, replacement of K with R |
|---|---|
| 235 | pcDNA3-myc-IgG HC (K235R) |
| 344 | pcDNA3-myc-IgG HC (K344R) |
| 431 | pcDNA3-myc-IgG HC (K431R) |

### 2. In vivo ubiquitination analysis

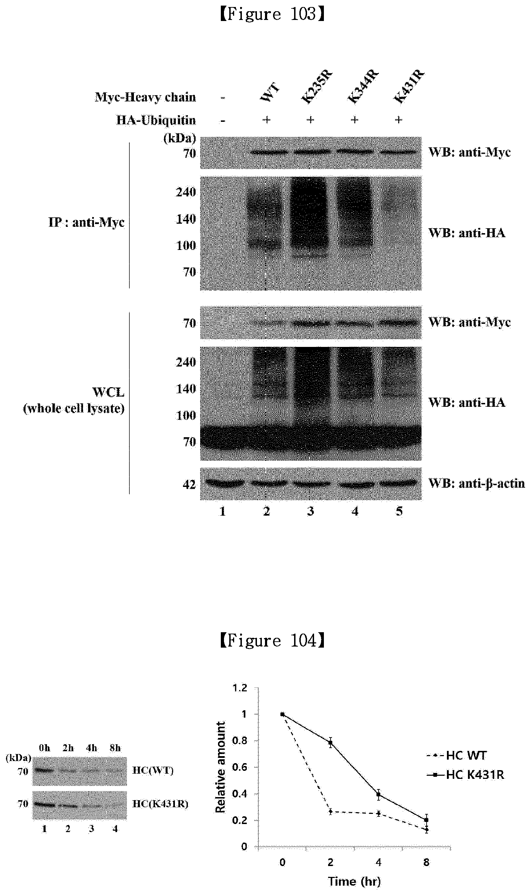

The HEK 293T cell was transfected with the plasmid encoding pcDNA3-myc-IgG-HC WT and pMT123-HA-ubiquitin. For the analysis of the ubiquitination level, pcDNA3-myc-IgG-HC WT 2 *µ*g and pMT123-HA-ubiquitin DNA 1 *µ*g were co-transfected into the cells. 24 hrs after the transfection, the cells were treated with MG132 (proteasome inhibitor, 5 *µ*g/mℓ) for 6 hrs, thereafter immunoprecipitation analysis was carried out (Fig. 102). Then, the HEK 293T cell was transfected with the plasmids encoding pcDNA3-myc-IgG-HC WT, pcDNA3-myc-IgG-HC mutant (K235R), pcDNA3-myc-IgG-HC mutant (K344R), pcDNA3-myc-IgG-HC mutant (K431R) and pMT123-HA-ubiquitin, respectively. For the analysis of the ubiquitination level, the cells were co-transfected with 1 *µ*g of pMT123-HA-ubiquitin DNA, and with respective 2 *µ*g of pcDNA3-myc-IgG-HC WT, pcDNA3-myc-IgG-HC mutant (K235R), pcDNA3-myc-IgG-HC mutant (K344R) and pcDNA3-myc-IgG-HC mutant (K431R). Next, 24 hrs after the transfection, immunoprecipitation was carried out (Fig. 103). The sample obtained for the immunoprecipitation was dissolved in buffering solution comprising (1% Triton X, 150 mM NaCl, 50 mM Tris-HCl, pH 8 and 1 mM PMSF (phenylmethanesulfonyl fluoride), and then was mixed with anti-myc (9E10) 1 st antibody (Santa Cruz Biotechnology, sc-40). Thereafter, the mixture was incubated at 4 °C, overnight. The immunoprecipitant was separated, following the reaction with A/G bead (Santa Cruz Biotechnology) at 4 °C, for 2 hrs. Subsequently, the separated immunoprecipitant was washed twice with buffering solution. The protein sample was separated by SDS-PAGE, after mixing with 2X SDS buffer and heating at 100 °C, for 7 minutes.

The separated protein was moved to polyvinylidene difluoride (PVDF) membrane, and then developed with ECL system using anti-mouse (Peroxidase-labeled antibody to mouse IgG (H+L), KPL, 074-1806) secondary antibody and blocking solution which comprises anti-myc (9E10, sc-40), anti-HA (sc-7392) and anti-β-actin (sc-47778) in 1:1,000 (w/w). As a result, when immunoprecipitation was performed by using anti-myc (9E10, sc-40), poly-ubiquitin chain was formed by the binding of the ubiquitin to pcDNA3-myc-IgG-HC WT, and thereby intense band indicating the presence of smear ubiquitin was detected (Fig. 102, lanes 3 and 4).

Further, when the cells were treated with MG132 (proteasome inhibitor, 5 *µ*g/mℓ) for 6 hrs, poly-ubiquitin chain formation was increased and thus the more intense band indicating ubiquitin was appeared (Fig. 102, lane 4). Further, as for the pcDNA3-myc-IgG-HC mutant (K431R), the band was less intense than the wild type, and smaller amount of ubiquitin was detected since the mutant above was not bound to the ubiquitin (Fig. 103, lane 5). These results represent that IgG-HC first binds to ubiquitin, and then is degraded through the polyubiquitin chain which is formed by ubiquitin-proteasome system.

### 3. Assessment of IgG -HC half-life using protein synthesis inhibitor cyclohexamide (CHX)

The HEK 293T cell was transfected with 2 *µ*g of pcDNA3-myc-IgG-HC WT, pcDNA3-myc-IgG-HC mutant (K235R), pcDNA3-myc-IgG-HC mutant (K344R) and pcDNA3-myc-IgG-HC mutant (K431R), respectively. 48 hrs after the transfection, the cells were treated with the protein synthesis inhibitor, cyclohexamide (CHX) (Sigma-Aldrich) (100 *µ*g/mℓ), and then the half-life of each protein was detected for 2, 4 and 8 hrs after the treatment of the inhibitor. As a result, the suppression of degradation of human IgG-HC was observed (Fig. 104). The half-life of human IgG-HC was less than 2 hrs, while the half-life of human pcDNA3-myc-IgG-HC mutant (K431R) was prolonged to 4 hrs or more, as shown in Fig. 104.

### Example 16: The analysis of ubiquitination and half-life increase of IgG light chain (LC), and the analysis of signal transduction in cells.

### 1. IgG light chain (LC) expression vector cloning and protein expression

### (1) IgG light chain (LC) expression vector cloning

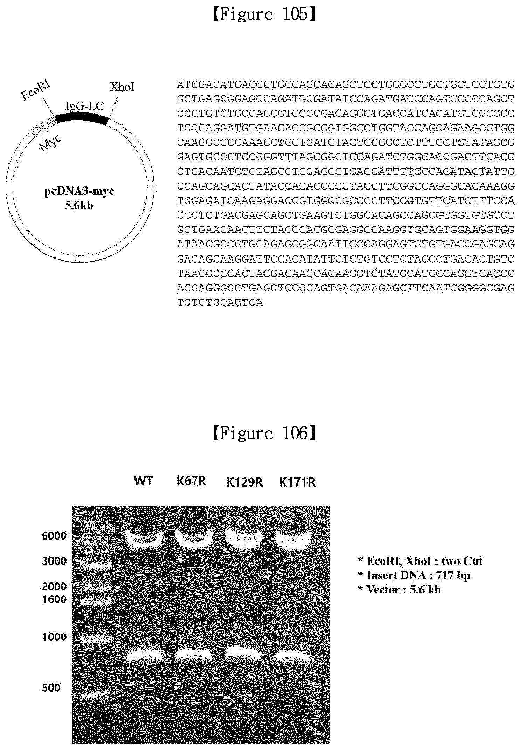

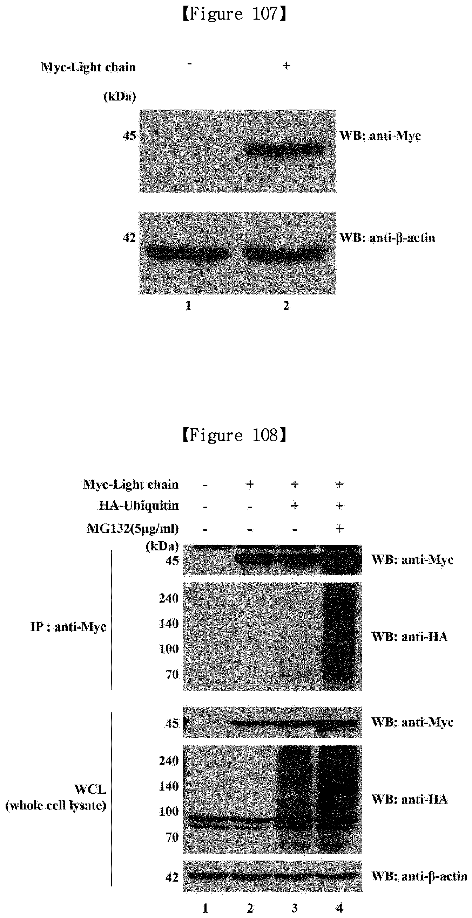

The IgG light chain (LC) DNA sequence was synthesized in accordance with the description of Roche's EP1308455 B9 (A composition comprising anti-HER2 antibodies, p. 23), and further optimized to express well in a mammalian cell. Then, IgG light chain (LC) DNA amplified by PCR was treated with EcoRI and XhoI, and then ligated to pcDNA3-myc vector (5.6kb) previously digested with the same enzyme (Fig. 105, IgG light chain amino acid sequence: SEQ No. 104). Then, agarose gel electrophoresis was carried out to confirm the presence of the DNA insert, after restriction enzyme digestion of the cloned vector (Fig. 106). For the assessment of the expression of proteins encoded by cloned DNA, western blot was carried out with anti-myc antibody (9E10, sc-40) to myc of pcDNA3-myc vector shown in the map of Fig. 105. The western blot result showed that the IgG light chain (LC) bound to myc was expressed well. The normalization with actin assured that proper amount of protein was loaded (Fig. 107).

### (2) Lysine (Lysine, K) residue substitution

Lysine residue was replaced with arginine (Arginine, R) using site-directed mutagenesis. The following primer sets were used for PCR to prepare the substituted plasmid DNAs.

(IgG LC K67R) FP 5'-CCTGGCAAGGCCCCAAGGCTGCTGATCTAC-3' (SEQ No. 105), RP 5'-GTAGATCAGCAGCCTTGGGGCCTTGCCAGG-3' (SEQ No. 106);

(IgG LC K129R) FP 5'-ACAAAGGTGGAGATCAGGAGGACCGTGGCC-3' (SEQ No. 107), RP 5'-GGCCACGGTCCTCCTGATCTCCACCTTTGT-3' (SEQ No. 108); and

(IgG LC K171R) FP 5'-GCCAAGGTGCAGTGGAGGGTGGATAACGCC-3' (SEQ No. 109), RP 5'-GGCGTTATCCACCCTCCACTGCACCTTGGC-3' (SEQ No. 110)

Three plasmid DNAs each of which one or more lysine residues were replaced with arginine (K→R) were prepared by using pcDNA3-myc-IgG LC DNA as a template (Table 16).

**[Table 16]**

| Lysine(K) residue site | IgG LC construct, replacement of K with R |
|---|---|
| 67 | pcDNA3-myc-IgG LC (K67R) |
| 129 | pcDNA3-myc-IgG LC (K129R) |
| 171 | pcDNA3-myc-IgG LC (K171R) |

### 2. In vivo ubiquitination analysis

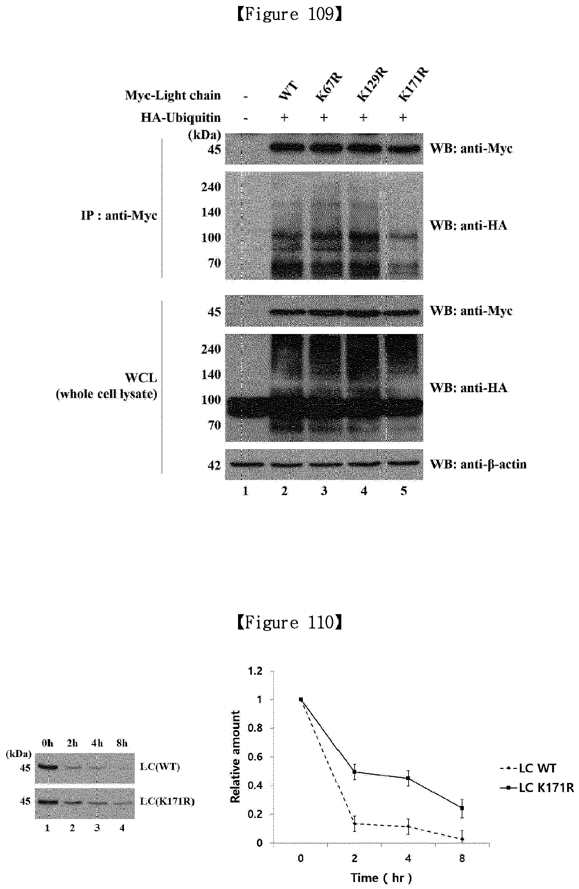

The HEK 293T cell was transfected with the plasmid encoding pcDNA3.1-6myc-IgG-LC WT and pMT123-HA-ubiquitin. For the analysis of the ubiquitination level, pcDNA3-myc-IgG-LC WT 2 *µ*g and pMT123-HA-ubiquitin DNA 1 *µ*g were co-transfected into the cells. 24 hrs after the transfection, the cells were treated with MG132 (proteasome inhibitor, 5 *µ*g/mℓ) for 6 hrs, thereafter immunoprecipitation analysis was carried out (Fig. 108). Then, the HEK 293T cells were transfected with the plasmids encoding pcDNA3-myc-IgG-LC WT, pcDNA3-myc-IgG-LC mutant (K67R), pcDNA3-myc-IgG-LC mutant (K129R), pcDNA3-myc-IgG-LC mutant (K171R) and pMT123-HA-ubiquitin, respectively. For the analysis of the ubiquitination level, the cells were co-transfected with 1 *µ*g of pMT123-HA-ubiquitin DNA, and with respective 2 *µ*g of pcDNA3-myc-IgG-LC WT, pcDNA3-myc-IgG-LC mutant (K67R), pcDNA3-myc-IgG-LC mutant (K129R) and pcDNA3-myc-IgG-LC mutant (K171R). Next, 24 hrs after the transfection, the immunoprecipitation was carried out (Fig. 109). The protein sample obtained for the immunoprecipitation was dissolved in buffering solution comprising (1% Triton X, 150 mM NaCl, 50 mM Tris-HCl, pH 8 and 1 mM PMSF (phenylmethanesulfonyl fluoride)), and then was mixed with anti-myc (9E10) 1 st antibody (Santa Cruz Biotechnology, sc-40). Thereafter, the mixture was incubated at 4 °C, overnight. The immunoprecipitant was separated, following the reaction with A/G bead (Santa Cruz Biotechnology) at 4 °C, for 2 hrs. Subsequently, the separated immunoprecipitant was washed twice with buffering solution. The protein sample was separated by SDS-PAGE, after mixing with 2X SDS buffer and heating at 100 °C, for 7 minutes. The separated proteins were moved to polyvinylidene difluoride (PVDF) membrane, and then developed with ECL system using anti-mouse (Peroxidase-labeled antibody to mouse IgG (H+L), KPL, 074-1806) secondary antibody and blocking solution which comprises anti-myc (9E10, sc-40), anti-HA (sc-7392) and anti-P-actin (sc-47778) in 1:1,000 (w/w). As a result, when immunoprecipitation was performed by using anti-myc (9E10, sc-40), poly-ubiquitin chain was formed by the binding of the ubiquitin to pcDNA3-myc-IgG-LC WT, and thereby intense band indicating the presence of smear ubiquitin was detected (Fig. 108, lanes 3 and 4). Further, when the cells were treated with MG132 (proteasome inhibitor, 5 *µ*g/mℓ) for 6 hrs, poly-ubiquitin chain formation was increased and thus the more intense band indicating ubiquitin was appeared (Fig. 108, lane 4). Further, as for the pcDNA3-myc-IgG-LC mutant (K171R), the band was less intense than the wild type, and smaller amount of ubiquitin was detected since the mutants above were not bound to the ubiquitin (Fig. 109, lane 5). These results represent that IgG-LC first binds to ubiquitin, and then is degraded through the polyubiquitin chain which is formed by ubiquitin-proteasome system.

### 3. Assessment of IgG -LC half-life using protein synthesis inhibitor cyclohexamide (CHX)

The HEK 293T cell was transfected with 2 *µ*g of pcDNA3-myc-IgG-LC WT, pcDNA3-myc-IgG-LC mutant (K67R), pcDNA3-myc-IgG-LC mutant (K129R) and pcDNA3-myc-IgG-LC mutant (K171R), respectively. 48 hrs after the transfection, the cells were treated with the protein synthesis inhibitor, cyclohexamide (CHX) (Sigma-Aldrich) (100 µg/mℓ), and then the half-life of the proteins was detected for 2, 4 and 8 hrs after the treatment of the inhibitor. As a result, the degradation of the substituted human IgG-LC of the present invention was suppressed (Fig. 110). The half-life of human IgG-LC was less than 1 hr, while the half-life of human pcDNA3-myc-IgG-LC mutant (K171R) was prolonged to 2 hrs or more, as shown in Fig. 110.

### Industrial Applicability

The present invention would be used to develop a protein or (poly)peptide therapeutic agents, since the mutated proteins of the invention have prolonged half-life.

## Claims

1. An immunoglobulin G (IgG), which comprises heavy chains (HC) and light chains (LC), having a prolonged half-life, wherein the IgG heavy chain (HC) and light chain (LC) has amino acid sequences of SEQ No. 94 and SEQ No. 101, respectively, and
wherein lysine residue(s) at position corresponding to 431 from the N-terminus of the IgG heavy chain (HC) and 171 from the N-terminus of the IgG light chain (LC) are replaced by arginine(s).

2. A pharmaceutical composition for preventing and/or treating immune deficiencies, autoimmune disorders, and infections, which comprises the IgG of claim 1, and pharmaceutically accepted excipient.

3. An expression vector comprising: (a) promoter; (b) a nucleic acid sequence encoding the IgG of claim 1; and optionally a linker, wherein the promoter and the nucleic acid sequence are operably linked.

4. A host cell comprising the expression vector of claim 3.

5. An immunoglobulin G (IgG) heavy chain (HC) having a prolonged half-life, wherein the IgG heavy chains (HC) has amino acid sequences of SEQ No. 94 and a lysine residue at position corresponding to 431 from the N-terminus of the IgG heavy chain (HC) is replaced by arginine(s).

6. An expression vector comprising: (a) promoter; (b) a nucleic acid sequence encoding the IgG heavy chain (HC) of claim 1; and optionally a linker, wherein the promoter and the nucleic acid sequence are operably linked.

7. A host cell comprising the expression vector of claim 6.

8. An immunoglobulin G (IgG) light chain (LC) having a prolonged half-life, wherein the IgG light chain (LC) has amino acid sequences of SEQ No. 101 and a lysine residue at position corresponding to 171 from the N-terminus of the IgG light chains (LC) is replaced by arginine(s).

9. An expression vector comprising: (a) promoter; (b) a nucleic acid sequence encoding the IgG light chain (LC) of claim 8; and optionally a linker, wherein the promoter and the nucleic acid sequence are operably linked.

10. A host cell comprising the expression vector of claim 9.

11. A host cell comprising the expression vectors of claim 6 and claim 9.


Sequence Listing

<110>    UbiProtein. Corp

<120>    A method for extending half-life of a protein

<130>    UBPRN16P01WO

<150>    KR 10-2015-0160728
<151>    2015-11-16

<160>    110

<170>    KoPatentIn 3.0

<210>    1
<211>    198
<212>    PRT
<213>    Artificial Sequence

<220>
<223>    Human beta trophin

<400>    1
Met Pro Val Pro Ala Leu Cys Leu Leu Trp Ala Leu Ala Met Val Thr
  1               5                  10                  15

Arg Pro Ala Ser Ala Ala Pro Met Gly Gly Pro Glu Leu Ala Gln His
             20                  25                  30

Glu Glu Leu Thr Leu Leu Phe His Gly Thr Leu Gln Leu Gly Gln Ala
         35                  40                  45

Leu Asn Gly Val Tyr Arg Thr Thr Glu Gly Arg Leu Thr Lys Ala Arg
     50                  55                  60

Asn Ser Leu Gly Leu Tyr Gly Arg Thr Ile Glu Leu Leu Gly Gln Glu
 65                  70                  75                  80

Val Ser Arg Gly Arg Asp Ala Ala Gln Glu Leu Arg Ala Ser Leu Leu
                 85                  90                  95

Glu Thr Gln Met Glu Glu Asp Ile Leu Gln Leu Gln Ala Glu Ala Thr
            100                 105                 110

Ala Glu Val Leu Gly Glu Val Ala Gln Ala Gln Lys Val Leu Arg Asp
        115                 120                 125

Ser Val Gln Arg Leu Glu Val Gln Leu Arg Ser Ala Trp Leu Gly Pro
    130                 135                 140

Ala Tyr Arg Glu Phe Glu Val Leu Lys Ala His Ala Asp Lys Gln Ser
145                 150                 155                 160

His Ile Leu Trp Ala Leu Thr Gly His Val Gln Arg Gln Arg Arg Glu
                165                 170                 175

Met Val Ala Gln Gln His Arg Leu Arg Gln Ile Gln Glu Arg Leu His
            180                 185                 190

Thr Ala Ala Leu Pro Ala
        195

<210>    2
<211>    25
<212>    DNA
<213>    Artificial Sequence

<220>
<223>    Human beta trophin

<400>    2
agggacggct gacaagggcc aggaa                                          25

<210>    3
<211>    26
<212>    DNA
<213>    Artificial Sequence

<220>
<223>    Human beta trophin

<400>    3
   ccaggctgtt cctggccctt gtcagc                                          26

<210>    4
<211>    25
<212>    DNA
<213>    Artificial Sequence

<220>
<223>    Human beta trophin

<400>    4
ggcacagagg gtgctacggg acagc                                            25

<210>    5
<211>    25
<212>    DNA
<213>    Artificial Sequence

<220>
<223>    Human beta trophin

<400>    5
cgtagcaccc tctgtgcctg ggcca                                            25

<210>    6
<211>    26
<212>    DNA
<213>    Artificial Sequence

<220>
<223>    Human beta trophin

<400>    6
gaatttgagg tcttaagggc tcacgc                                          26

<210>    7
<211>    27
<212>    DNA
<213>    Artificial Sequence

<220>
<223>    Human beta trophin

<400>    7
cttgtcagcg tgagccctta agacctc                                         27

<210>    8
<211>    26
<212>    DNA
<213>    Artificial Sequence

<220>
<223>    Human beta trophin

<400>    8
gctcacgctg acaggcagag ccacat                                          26

<210>    9
<211>    27
<212>    DNA
<213>    Artificial Sequence

<220>

<223>    Human beta trophin

<400>    9
ccataggatg tggctctgcc tgtcagc                                          27

<210>    10
<211>    217
<212>    PRT
<213>    Artificial Sequence

<220>
<223>    Human growth hormone

<400>    10

<210>    11
<211>    24
<212>    DNA
<213>    Artificial Sequence

<220>
<223>    Human growth hormone

<400>    11
ccaaaggaac agaggtattc attc                                              24

<210>    12
<211>    24
<212>    DNA
<213>    Artificial Sequence

<220>
<223>    Human growth hormone

<400>    12
caggaatgaa tacctctgtt cctt                                              24

<210>    13
<211>    21
<212>    DNA
<213>    Artificial Sequence

<220>
<223>    Human growth hormone

<400>    13
gacctcctaa gggacctaga g                                                 21

<210>    14
<211>    21
<212>    DNA
<213>    Artificial Sequence

<220>
<223>    Human growth hormone

<400>    14
ctctaggtcc cttaggaggt c                                                 21

<210>    15
<211>    21
<212>    DNA
<213>    Artificial Sequence

<220>
<223>    Human growth hormone

<400>    15
cagatcttca ggcagaccta c                                                21

<210>    16
<211>    21
<212>    DNA
<213>    Artificial Sequence

<220>
<223>    Human growth hormone

<400>    16
gtaggtctgc ctgaagatct g                                                21

<210>    17
<211>    110
<212>    PRT
<213>    Artificial Sequence

<220>
<223>    Human insulin

<400>    17

<210>    18
<211>    25
<212>    DNA
<213>    Artificial Sequence

<220>
<223>    Human insulin

<400>    18
ggcttcttct acacacccag gaccc                                          25

<210>    19
<211>    24
<212>    DNA
<213>    Artificial Sequence

<220>
<223>    Human insulin

<400>    19
ctcccggcgg gtcctgggtg tgta                                           24

<210>    20
<211>    23
<212>    DNA
<213>    Artificial Sequence

<220>
<223>    Human insulin

<400>    20
tccctgcaga ggcgtggcat tgt                                              23

<210>    21
<211>    27
<212>    DNA
<213>    Artificial Sequence

<220>
<223>    Human insulin

<400>    21
ttgttccaca atgccacgcc tctgcag                                          27

<210>    22
<211>    188
<212>    PRT
<213>    Artificial Sequence

<220>
<223>    Human interferon alpha

<400>    22
Met Ala Leu Thr Phe Ala Leu Leu Val Ala Leu Leu Val Leu Ser Cys
1               5                   10                  15

Lys Ser Ser Cys Ser Val Gly Cys Asp Leu Pro Gln Thr His Ser Leu
            20                  25                  30

Gly Ser Arg Arg Thr Leu Met Leu Leu Ala Gln Met Arg Arg Ile Ser
        35                  40                  45

Leu Phe Ser Cys Leu Lys Asp Arg His Asp Phe Gly Phe Pro Gln Glu
50                  55                  60

Glu Phe Gly Asn Gln Phe Gln Lys Ala Glu Thr Ile Pro Val Leu His
65                  70                  75                  80

Glu Met Ile Gln Gln Ile Phe Asn Leu Phe Ser Thr Lys Asp Ser Ser
                85                  90                  95

Ala Ala Trp Asp Glu Thr Leu Leu Asp Lys Phe Tyr Thr Glu Leu Tyr
            100                 105                 110

Gln Gln Leu Asn Asp Leu Glu Ala Cys Val Ile Gln Gly Val Gly Val
        115                 120                 125

Thr Glu Thr Pro Leu Met Lys Glu Asp Ser Ile Leu Ala Val Arg Lys
    130                 135                 140

Tyr Phe Gln Arg Ile Thr Leu Tyr Leu Lys Glu Lys Lys Tyr Ser Pro
145                 150                 155                 160

Cys Ala Trp Glu Val Val Arg Ala Glu Ile Met Arg Ser Phe Ser Leu
                165                 170                 175

Ser Thr Asn Leu Gln Glu Ser Leu Arg Ser Lys Glu
            180                 185

<210>    23
<211>    21
<212>    DNA
<213>    Artificial Sequence

<220>
<223>    Human interferon alpha

<400>    23
cttcagcaca agggactcat c                                                21

<210>    24
<211>    21
<212>    DNA
<213>    Artificial Sequence

<220>
<223>    Human interferon alpha

<400>    24
cagatgagtc ccttgtgctg a                                                21

<210>    25
<211>    21
<212>    DNA
<213>    Artificial Sequence

<220>
<223>    Human interferon alpha

<400>    25
ctcctagaca gattctacac t                                                21

<210>    26
<211>    21
<212>    DNA
<213>    Artificial Sequence

<220>
<223>    Human interferon alpha

<400>    26
agtgtagaat ctgtctagga g                                                21

<210>    27

<211>    21

<212>    DNA

<213>    Artificial Sequence

<220>

<223>    Human interferon alpha

<400>    27

gctgtgagga gatacttcca a                                                21

<210>    28

<211>    21

<212>    DNA

<213>    Artificial Sequence

<220>

<223>    Human interferon alpha

<400>    28

ttggaagtat ctcctcacag c                                                21

<210>    29

<211>    21

<212>    DNA

<213>    Artificial Sequence

<220>

<223>    Human interferon alpha

<400>    29

ctctatctga gagagaagaa a                                                21

<210>    30
<211>    21
<212>    DNA
<213>    Artificial Sequence

<220>
<223>    Human interferon alpha

<400>    30
tttcttctct ctcagataga g                                                    21

<210>    31
<211>    207
<212>    PRT
<213>    Artificial Sequence

<220>
<223>    Human G-CSF

<400>    31
Met Ala Gly Pro Ala Thr Gln Ser Pro Met Lys Leu Met Ala Leu Gln
1               5                   10                  15

Leu Leu Leu Trp His Ser Ala Leu Trp Thr Val Gln Glu Ala Thr Pro
            20                  25                  30

Leu Gly Pro Ala Ser Ser Leu Pro Gln Ser Phe Leu Leu Lys Cys Leu
        35                  40                  45

Glu Gln Val Arg Lys Ile Gln Gly Asp Gly Ala Ala Leu Gln Glu Lys
    50                  55                  60

Leu Val Ser Glu Cys Ala Thr Tyr Lys Leu Cys His Pro Glu Glu Leu
65                  70                  75                  80

Val Leu Leu Gly His Ser Leu Gly Ile Pro Trp Ala Pro Leu Ser Ser
                85                  90                  95

Cys Pro Ser Gln Ala Leu Gln Leu Ala Gly Cys Leu Ser Gln Leu His
            100                 105                 110

Ser Gly Leu Phe Leu Tyr Gln Gly Leu Leu Gln Ala Leu Glu Gly Ile
        115                 120                 125

Ser Pro Glu Leu Gly Pro Thr Leu Asp Thr Leu Gln Leu Asp Val Ala
    130                 135                 140

Asp Phe Ala Thr Thr Ile Trp Gln Gln Met Glu Glu Leu Gly Met Ala
145                 150                 155                 160

Pro Ala Leu Gln Pro Thr Gln Gly Ala Met Pro Ala Phe Ala Ser Ala
                165                 170                 175

Phe Gln Arg Arg Ala Gly Gly Val Leu Val Ala Ser His Leu Gln Ser
            180                 185                 190

Phe Leu Glu Val Ser Tyr Arg Val Leu Arg His Leu Ala Gln Pro
        195                 200                 205

<210>    32
<211>    24
<212>    DNA
<213>    Artificial Sequence

<220>
<223>    Human G-CSF

<400>    32
agcttcctgc tcaggtgctt agag                                              24

<210>    33
<211>    24

<212>    DNA
<213>    Artificial Sequence

<220>
<223>    Human G-CSF

<400>    33
ttgctctaag cacctgagca ggaa                                              24

<210>    34
<211>    24
<212>    DNA
<213>    Artificial Sequence

<220>
<223>    Human G-CSF

<400>    34
tgtgccacct acaggctgtg ccac                                              24

<210>    35
<211>    24
<212>    DNA
<213>    Artificial Sequence

<220>
<223>    Human G-CSF

<400>    35
ggggtggcac agcctgtagg tggc                                              24

<210>    36
<211>    187
<212>    PRT

<213>    Artificial Sequence

<220>
<223>    Human interferon beta

<400>    36
Met Thr Asn Lys Cys Leu Leu Gln Ile Ala Leu Leu Leu Cys Phe Ser
1               5                   10                  15

Thr Thr Ala Leu Ser Met Ser Tyr Asn Leu Leu Gly Phe Leu Gln Arg
            20                  25                  30

Ser Ser Asn Phe Gln Cys Gln Lys Leu Leu Trp Gln Leu Asn Gly Arg
        35                  40                  45

Leu Glu Tyr Cys Leu Lys Asp Arg Met Asn Phe Asp Ile Pro Glu Glu
    50                  55                  60

Ile Lys Gln Leu Gln Gln Phe Gln Lys Glu Asp Ala Ala Leu Thr Ile
65                  70                  75                  80

Tyr Glu Met Leu Gln Asn Ile Phe Ala Ile Phe Arg Gln Asp Ser Ser
                85                  90                  95

Ser Thr Gly Trp Asn Glu Thr Ile Val Glu Asn Leu Leu Ala Asn Val
            100                 105                 110

Tyr His Gln Ile Asn His Leu Lys Thr Val Leu Glu Glu Lys Leu Glu
        115                 120                 125

Lys Glu Asp Phe Thr Arg Gly Lys Leu Met Ser Ser Leu His Leu Lys
    130                 135                 140

Arg Tyr Tyr Gly Arg Ile Leu His Tyr Leu Lys Ala Lys Glu Tyr Ser
145                 150                 155                 160

His Cys Ala Trp Thr Ile Val Arg Val Glu Ile Leu Arg Asn Phe Tyr
                165                 170                 175

Phe Ile Asn Arg Leu Thr Gly Tyr Leu Arg Asn
            180                 185

<210>    37
<211>    21
<212>    DNA
<213>    Artificial Sequence

<220>
<223>    Human interferon beta

<400>    37
cagtgtcaga ggctcctgtg g                                                    21

<210>    38
<211>    21
<212>    DNA
<213>    Artificial Sequence

<220>
<223>    Human interferon beta

<400>    38
ccacaggagc ctctgacact g                                                    21

<210>    39
<211>    21
<212>    DNA
<213>    Artificial Sequence

<220>
<223>    Human interferon beta

<400>    39

ctggaagaaa gactggagaa a                                                21

<210>    40
<211>    21
<212>    DNA
<213>    Artificial Sequence

<220>
<223>    Human interferon beta

<400>    40
tttctccagt ctttcttcca g                                                21

<210>    41
<211>    21
<212>    DNA
<213>    Artificial Sequence

<220>
<223>    Human interferon beta

<400>    41
cattacctga gggccaagga g                                                21

<210>    42
<211>    21
<212>    DNA
<213>    Artificial Sequence

<220>
<223>    Human interferon beta

<400>    42
ctccttggcc ctcaggtaat g                                                21

<210> 43
<211> 193
<212> PRT
<213> Artificial Sequence

<220>
<223> Human erythropoietin

<400> 43
Met Gly Val His Glu Cys Pro Ala Trp Leu Trp Leu Leu Leu Ser Leu
1               5                   10                  15

Leu Ser Leu Pro Leu Gly Leu Pro Val Leu Gly Ala Pro Pro Arg Leu
            20                  25                  30

Ile Cys Asp Ser Arg Val Leu Glu Arg Tyr Leu Leu Glu Ala Lys Glu
        35                  40                  45

Ala Glu Asn Ile Thr Thr Gly Cys Ala Glu His Cys Ser Leu Asn Glu
    50                  55                  60

Asn Ile Thr Val Pro Asp Thr Lys Val Asn Phe Tyr Ala Trp Lys Arg
65                  70                  75                  80

Met Glu Val Gly Gln Gln Ala Val Glu Val Trp Gln Gly Leu Ala Leu
                85                  90                  95

Leu Ser Glu Ala Val Leu Arg Gly Gln Ala Leu Leu Val Asn Ser Ser
            100                 105                 110

Gln Pro Trp Glu Pro Leu Gln Leu His Val Asp Lys Ala Val Ser Gly
        115                 120                 125

Leu Arg Ser Leu Thr Thr Leu Leu Arg Ala Leu Gly Ala Gln Lys Glu
    130                 135                 140

Ala Ile Ser Pro Pro Asp Ala Ala Ser Ala Ala Pro Leu Arg Thr Ile

145                 150                 155                 160

Thr Ala Asp Thr Phe Arg Lys Leu Phe Arg Val Tyr Ser Asn Phe Leu
                165                 170                 175

Arg Gly Lys Leu Lys Leu Tyr Thr Gly Glu Ala Cys Arg Thr Gly Asp
            180                 185                 190

Arg

<210>    44
<211>    24
<212>    DNA
<213>    Artificial Sequence

<220>
<223>    Human erythropoietin

<400>    44
gcatgtggat agagccgtca gtgc                                              24

<210>    45
<211>    24
<212>    DNA
<213>    Artificial Sequence

<220>
<223>    Human erythropoietin

<400>    45
gcactgacgg ctctatccac atgc                                              24

<210>    46
<211>    31

<212>    DNA
<213>    Artificial Sequence

<220>
<223>    Human erythropoietin

<400>    46
tgacactttc cgcagactct tccgagtcta c                                    31

<210>    47
<211>    31
<212>    DNA
<213>    Artificial Sequence

<220>
<223>    Human erythropoietin

<400>    47
gtagactcgg aagagtctgc ggaaagtgtc a                                    31

<210>    48
<211>    21
<212>    DNA
<213>    Artificial Sequence

<220>
<223>    Human erythropoietin

<400>    48
ctccggggaa ggctgaagct g                                               21

<210>    49
<211>    21
<212>    DNA

<213>    Artificial Sequence

<220>
<223>    Human erythropoietin

<400>    49
cagcttcagc cttccccgga g                                                21

<210>    50
<211>    23
<212>    DNA
<213>    Artificial Sequence

<220>
<223>    Human erythropoietin

<400>    50
ggaaagctga ggctgtacac agg                                              23

<210>    51
<211>    23
<212>    DNA
<213>    Artificial Sequence

<220>
<223>    Human erythropoietin

<400>    51
cctgtgtaca gcctcagctt tcc                                              23

<210>    52
<211>    396
<212>    PRT
<213>    Artificial Sequence

<220>
<223>    Human bone morphogenetic protein-2

<400>    52
Met Val Ala Gly Thr Arg Cys Leu Leu Ala Leu Leu Leu Pro Gln Val
1               5                   10                  15

Leu Leu Gly Gly Ala Ala Gly Leu Val Pro Glu Leu Gly Arg Arg Lys
            20                  25                  30

Phe Ala Ala Ala Ser Ser Gly Arg Pro Ser Ser Gln Pro Ser Asp Glu
        35                  40                  45

Val Leu Ser Glu Phe Glu Leu Arg Leu Leu Ser Met Phe Gly Leu Lys
    50                  55                  60

Gln Arg Pro Thr Pro Ser Arg Asp Ala Val Val Pro Pro Tyr Met Leu
65                  70                  75                  80

Asp Leu Tyr Arg Arg His Ser Gly Gln Pro Gly Ser Pro Ala Pro Asp
                85                  90                  95

His Arg Leu Glu Arg Ala Ala Ser Arg Ala Asn Thr Val Arg Ser Phe
            100                 105                 110

His His Glu Glu Ser Leu Glu Glu Leu Pro Glu Thr Ser Gly Lys Thr
        115                 120                 125

Thr Arg Arg Phe Phe Phe Asn Leu Ser Ser Ile Pro Thr Glu Glu Phe
    130                 135                 140

Ile Thr Ser Ala Glu Leu Gln Val Phe Arg Glu Gln Met Gln Asp Ala
145                 150                 155                 160

Leu Gly Asn Asn Ser Ser Phe His His Arg Ile Asn Ile Tyr Glu Ile
                165                 170                 175

Ile Lys Pro Ala Thr Ala Asn Ser Lys Phe Pro Val Thr Arg Leu Leu

            180                 185                 190

Asp Thr Arg Leu Val Asn Gln Asn Ala Ser Arg Trp Glu Ser Phe Asp
        195                 200                 205

Val Thr Pro Ala Val Met Arg Trp Thr Ala Gln Gly His Ala Asn His
    210                 215                 220

Gly Phe Val Val Glu Val Ala His Leu Glu Glu Lys Gln Gly Val Ser
225                 230                 235                 240

Lys Arg His Val Arg Ile Ser Arg Ser Leu His Gln Asp Glu His Ser
                245                 250                 255

Trp Ser Gln Ile Arg Pro Leu Leu Val Thr Phe Gly His Asp Gly Lys
            260                 265                 270

Gly His Pro Leu His Lys Arg Glu Lys Arg Gln Ala Lys His Lys Gln
        275                 280                 285

Arg Lys Arg Leu Lys Ser Ser Cys Lys Arg His Pro Leu Tyr Val Asp
    290                 295                 300

Phe Ser Asp Val Gly Trp Asn Asp Trp Ile Val Ala Pro Pro Gly Tyr
305                 310                 315                 320

His Ala Phe Tyr Cys His Gly Glu Cys Pro Phe Pro Leu Ala Asp His
                325                 330                 335

Leu Asn Ser Thr Asn His Ala Ile Val Gln Thr Leu Val Asn Ser Val
            340                 345                 350

Asn Ser Lys Ile Pro Lys Ala Cys Cys Val Pro Thr Glu Leu Ser Ala
        355                 360                 365

Ile Ser Met Leu Tyr Leu Asp Glu Asn Glu Lys Val Val Leu Lys Asn
    370                 375                 380

Tyr Gln Asp Met Val Val Glu Gly Cys Gly Cys Arg
385                 390                 395

<210> 53
<211> 29
<212> DNA
<213> Artificial Sequence

<220>
<223> Human bone morphogenetic protein-2

<400> 53
gaaacgcctt aggtccagct gtaagagac 29

<210> 54
<211> 29
<212> DNA
<213> Artificial Sequence

<220>
<223> Human bone morphogenetic protein-2

<400> 54
gtctcttaca gctggaccta aggcgtttc 29

<210> 55
<211> 30
<212> DNA
<213> Artificial Sequence

<220>
<223> Human bone morphogenetic protein-2

<400> 55
ttaagtccag ctgtaggaga caccctttgt 30

<210> 56
<211> 30
<212> DNA
<213> Artificial Sequence

<220>
<223> Human bone morphogenetic protein-2

<400> 56
acaaagggtg tctcctacag ctggacttaa 30

<210> 57
<211> 23
<212> DNA
<213> Artificial Sequence

<220>
<223> Human bone morphogenetic protein-2

<400> 57
gttaactcta ggattcctaa ggc 23

<210> 58
<211> 23
<212> DNA
<213> Artificial Sequence

<220>
<223> Human bone morphogenetic protein-2

<400> 58
gccttaggaa tcctagagtt aac 23

<210>    59
<211>    25
<212>    DNA
<213>    Artificial Sequence

<220>
<223>    Human bone morphogenetic protein-2

<400>    59
ggttgtatta aggaactatc aggac                                              25

<210>    60
<211>    25
<212>    DNA
<213>    Artificial Sequence

<220>
<223>    Human bone morphogenetic protein-2

<400>    60
gtcctgatag ttccttaata caacc                                              25

<210>    61
<211>    155
<212>    PRT
<213>    Artificial Sequence

<220>
<223>    Human fibroblast growth factor-1

<400>    61
Met Ala Glu Gly Glu Ile Thr Thr Phe Thr Ala Leu Thr Glu Lys Phe
 1               5                  10                  15

Asn Leu Pro Pro Gly Asn Tyr Lys Lys Pro Lys Leu Leu Tyr Cys Ser

20                  25                  30

Asn Gly Gly His Phe Leu Arg Ile Leu Pro Asp Gly Thr Val Asp Gly
        35                  40                  45

Thr Arg Asp Arg Ser Asp Gln His Ile Gln Leu Gln Leu Ser Ala Glu
    50                  55                  60

Ser Val Gly Glu Val Tyr Ile Lys Ser Thr Glu Thr Gly Gln Tyr Leu
65                  70                  75                  80

Ala Met Asp Thr Asp Gly Leu Leu Tyr Gly Ser Gln Thr Pro Asn Glu
                85                  90                  95

Glu Cys Leu Phe Leu Glu Arg Leu Glu Glu Asn His Tyr Asn Thr Tyr
            100                 105                 110

Ile Ser Lys Lys His Ala Glu Lys Asn Trp Phe Val Gly Leu Lys Lys
        115                 120                 125

Asn Gly Ser Cys Lys Arg Gly Pro Arg Thr His Tyr Gly Gln Lys Ala
    130                 135                 140

Ile Leu Phe Leu Pro Leu Pro Val Ser Ser Asp
145                 150                 155

<210>    62
<211>    21
<212>    DNA
<213>    Artificial Sequence

<220>
<223>    Human fibroblast growth factor-1

<400>    62
aagaagccca gactcctcta c                                                21

<210> 63
<211> 21
<212> DNA
<213> Artificial Sequence

<220>
<223> Human fibroblast growth factor-1

<400> 63
gtagaggagt ctgggcttct t 21

<210> 64
<211> 21
<212> DNA
<213> Artificial Sequence

<220>
<223> Human fibroblast growth factor-1

<400> 64
catgcagaga ggaattggtt t 21

<210> 65
<211> 21
<212> DNA
<213> Artificial Sequence

<220>
<223> Human fibroblast growth factor-1

<400> 65
aaaccaattc ctctctgcat g 21

<210> 66

<211>    167

<212>    PRT

<213>    Artificial Sequence

<220>

<223>    Human Leptin

<400>    66

<210> 67
<211> 21
<212> DNA
<213> Artificial Sequence

<220>
<223> Human Leptin

<400> 67
cccatccaaa aggtccaaga t 21

<210> 68
<211> 21
<212> DNA
<213> Artificial Sequence

<220>
<223> Human Leptin

<400> 68
atcttggacc ttttggatgg g 21

<210> 69
<211> 21
<212> DNA
<213> Artificial Sequence

<220>
<223> Human Leptin

<400> 69
gatgacacca agaccctcat c 21

<210> 70
<211> 21
<212> DNA
<213> Artificial Sequence

<220>
<223> Human Leptin

<400> 70
gatgagggtc ttggtgtcat c 21

<210> 71
<211> 21
<212> DNA
<213> Artificial Sequence

<220>
<223> Human Leptin

<400> 71
accctcatca ggacaattgt c 21

<210> 72
<211> 21
<212> DNA
<213> Artificial Sequence

<220>
<223> Human Leptin

<400> 72
gacaattgtc ctgatgaggg t 21

<210> 73
<211> 21
<212> DNA
<213> Artificial Sequence

<220>
<223> Human Leptin

<400> 73
accttatcca ggatggacca g 21

<210> 74
<211> 21
<212> DNA
<213> Artificial Sequence

<220>
<223> Human Leptin

<400> 74
ctggtccatc ctggataagg t 21

<210> 75
<211> 209
<212> PRT
<213> Artificial Sequence

<220>
<223> Human vascular endothelial growth factor A

<400> 75
Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
        35                  40                  45

Arg Ser Tyr Cys His Pro Ile Glu Thr Leu Val Asp Ile Phe Gln Glu
    50                  55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
            100                 105                 110

Gln Gly Gln His Ile Gly Glu Met Ser Phe Leu Gln His Asn Lys Cys
        115                 120                 125

Glu Cys Arg Pro Lys Lys Asp Arg Ala Arg Gln Glu Lys Lys Ser Val
    130                 135                 140

Arg Gly Lys Gly Lys Gly Gln Lys Arg Lys Arg Lys Lys Ser Arg Pro
145                 150                 155                 160

Cys Gly Pro Cys Ser Glu Arg Arg Lys His Leu Phe Val Gln Asp Pro
                165                 170                 175

Gln Thr Cys Lys Cys Ser Cys Lys Asn Thr Asp Ser Arg Cys Lys Ala
            180                 185                 190

Arg Gln Leu Glu Leu Asn Glu Arg Thr Cys Arg Cys Asp Lys Pro Arg
        195                 200                 205

Arg

<210>    76
<211>    28
<212>    DNA
<213>    Artificial Sequence

<220>
<223>    Human vascular endothelial growth factor A

<400>    76
tacagcacaa cagatgtgaa tgcagacc                                          28

<210>    77
<211>    28
<212>    DNA
<213>    Artificial Sequence

<220>
<223>    Human vascular endothelial growth factor A

<400>    77
ggtctgcatt cacatctgtt gtgctgta                                          28

<210>    78
<211>    27
<212>    DNA
<213>    Artificial Sequence

<220>
<223>    Human vascular endothelial growth factor A

<400>    78
atccgcagac gtgtagatgt tcctgca                                           27

<210>    79

<211>    27
<212>    DNA
<213>    Artificial Sequence

<220>
<223>    Human vascular endothelial growth factor A

<400>    79
tgcaggaaca tctacacgtc tgcggat                                          27

<210>    80
<211>    117
<212>    PRT
<213>    Artificial Sequence

<220>
<223>    Human prepro-GHRL

<400>    80
Met Pro Ser Pro Gly Thr Val Cys Ser Leu Leu Leu Leu Gly Met Leu
1               5                   10                  15

Trp Leu Asp Leu Ala Met Ala Gly Ser Ser Phe Leu Ser Pro Glu His
            20                  25                  30

Gln Arg Val Gln Gln Arg Lys Glu Ser Lys Lys Pro Pro Ala Lys Leu
        35                  40                  45

Gln Pro Arg Ala Leu Ala Gly Trp Leu Arg Pro Glu Asp Gly Gly Gln
    50                  55                  60

Ala Glu Gly Ala Glu Asp Glu Met Glu Val Arg Phe Asn Ala Pro Phe
65                  70                  75                  80

Asp Val Gly Ile Lys Leu Ser Gly Val Gln Tyr Gln Gln His Ser Gln
                85                  90                  95

Ala Leu Gly Lys Phe Leu Gln Asp Ile Leu Trp Glu Glu Ala Lys Glu
            100                 105                 110

Ala Pro Ala Asp Lys
        115

<210>    81
<211>    21
<212>    DNA
<213>    Artificial Sequence

<220>
<223>    Human prepro-GHRL

<400>    81
gccctgggga ggtttcttca g                                                21

<210>    82
<211>    21
<212>    DNA
<213>    Artificial Sequence

<220>
<223>    Human prepro-GHRL

<400>    82
ctgaagaaac ctccccaggg c                                                21

<210>    83
<211>    28
<212>    PRT
<213>    Artificial Sequence

<220>
<223>    Human appetite stimulating hormone (Ghrelin)

<400>    83
Gly Ser Ser Phe Leu Ser Pro Glu His Gln Arg Val Gln Gln Arg Lys
1               5                   10                  15

Glu Ser Lys Lys Pro Pro Ala Lys Leu Gln Pro Arg
            20                  25

<210>    84
<211>    31
<212>    DNA
<213>    Artificial Sequence

<220>
<223>    Human appetite stimulating hormone (Ghrelin)

<400>    84
agtccagcag agaagggagt cgaagaagcc a                                    31

<210>    85
<211>    31
<212>    DNA
<213>    Artificial Sequence

<220>
<223>    Human appetite stimulating hormone (Ghrelin)

<400>    85
tggcttcttc gactcccttc tctgctggac t                                    31

<210>    86
<211>    31
<212>    DNA
<213>    Artificial Sequence

<220>
<223>    Human appetite stimulating hormone (Ghrelin)

<400>    86
agaaaggagt cgaggaagcc accagccaag c                                    31

<210>    87
<211>    31
<212>    DNA
<213>    Artificial Sequence

<220>
<223>    Human appetite stimulating hormone (Ghrelin)

<400>    87
gcttggctgg tggcttcctc gactcctttc t                                    31

<210>    88
<211>    31
<212>    DNA
<213>    Artificial Sequence

<220>
<223>    Human appetite stimulating hormone (Ghrelin)

<400>    88
agaaaggagt cgaagaggcc accagccaag c                                    31

<210>    89
<211>    31
<212>    DNA
<213>    Artificial Sequence

<220>
<223>    Human appetite stimulating hormone (Ghrelin)

<400>    89
gcttggctgg tggcctcttc gactcctttc t                                    31

<210>    90
<211>    30
<212>    DNA
<213>    Artificial Sequence

<220>
<223>    Human appetite stimulating hormone (Ghrelin)

<400>    90
aagaagccac cagccaggct gcagccccga                                      30

<210>    91
<211>    30
<212>    DNA
<213>    Artificial Sequence

<220>
<223>    Human appetite stimulating hormone (Ghrelin)

<400>    91
tcggggctgc agcctggctg gtggcttctt                                      30

<210>    92
<211>    31
<212>    PRT
<213>    Artificial Sequence

<220>

<223>    Human glucagon-like peptide-1 (GLP-1)

<400>    92
His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>    93
<211>    18
<212>    DNA
<213>    Artificial Sequence

<220>
<223>    Human glucagon-like peptide-1 (GLP-1)

<400>    93
aagctgccag ggaattca                                                    18

<210>    94
<211>    18
<212>    DNA
<213>    Artificial Sequence

<220>
<223>    Human glucagon-like peptide-1 (GLP-1)

<400>    94
tgaattccct ggcagctt                                                    18

<210>    95
<211>    17

<212>    DNA
<213>    Artificial Sequence

<220>
<223>    Human glucagon-like peptide-1 (GLP-1)

<400>    95
ttggctggtg agaggcc                                                    17

<210>    96
<211>    17
<212>    DNA
<213>    Artificial Sequence

<220>
<223>    Human glucagon-like peptide-1 (GLP-1)

<400>    96
ggcctctcac cagccaa                                                    17

<210>    97
<211>    470
<212>    PRT
<213>    Artificial Sequence

<220>
<223>    Human IgG heavy chain

<400>    97
Met Asp Trp Thr Trp Arg Phe Leu Phe Val Val Ala Ala Ala Thr Gly
1               5                   10                  15

Val Gln Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Asn Ile
        35                  40                  45

Lys Asp Thr Tyr Ile His Trp Val Arg Gln Ala Pro Gly Lys Gly Leu
    50                  55                  60

Glu Trp Val Ala Arg Ile Tyr Pro Thr Asn Gly Tyr Thr Arg Tyr Ala
65                  70                  75                  80

Asp Ser Val Lys Gly Arg Phe Thr Ile Ser Ala Asp Thr Ser Lys Asn
                85                  90                  95

Thr Ala Tyr Leu Gln Met Asn Ser Leu Arg Ala Glu Asp Thr Ala Val
            100                 105                 110

Tyr Tyr Cys Ser Arg Trp Gly Gly Asp Gly Phe Tyr Ala Met Asp Tyr
        115                 120                 125

Trp Gly Gln Gly Thr Leu Val Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu

245                 250                 255

Leu Gly Gly Pro Ser Val Phe Leu Phe Pro Pro Lys Pro Lys Asp Thr
        260                 265                 270

Leu Met Ile Ser Arg Thr Pro Glu Val Thr Cys Val Val Val Asp Val
    275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
        340                 345                 350

Pro Ile Glu Lys Thr Ile Ser Lys Ala Lys Gly Gln Pro Arg Glu Pro
    355                 360                 365

Gln Val Tyr Thr Leu Pro Pro Ser Arg Glu Glu Met Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
        420                 425                 430

Thr Val Asp Lys Ser Arg Trp Gln Gln Gly Asn Val Phe Ser Cys Ser
    435                 440                 445

Val Met His Glu Ala Leu His Asn His Tyr Thr Gln Lys Ser Leu Ser
    450                 455                 460

Leu Ser Pro Gly Leu Glu
465                 470

<210>    98
<211>    30
<212>    DNA
<213>    Artificial Sequence

<220>
<223>    Human IgG heavy chain

<400>    98
acaaaggtgg acaggaaggt ggagcccaag                                        30

<210>    99
<211>    30
<212>    DNA
<213>    Artificial Sequence

<220>
<223>    Human IgG heavy chain

<400>    99
cttgggctcc accttcctgt ccacctttgt                                        30

<210>    100
<211>    34
<212>    DNA
<213>    Artificial Sequence

<220>
<223>    Human IgG heavy chain

<400>    100
gagtataagt gcagggtgtc caataaggcc ctgc                                    34

<210>    101
<211>    34
<212>    DNA
<213>    Artificial Sequence

<220>
<223>    Human IgG heavy chain

<400>    101
gcagggcctt attggacacc ctgcacttat actc                                    34

<210>    102
<211>    34
<212>    DNA
<213>    Artificial Sequence

<220>
<223>    Human IgG heavy chain

<400>    102
ctttctgtat agcaggctga ccgtggataa gtcc                                    34

<210>    103
<211>    34
<212>    DNA
<213>    Artificial Sequence

<220>
<223>    Human IgG heavy chain

<400>    103

ggacttatcc acggtcagcc tgctatacag aaag                                    34

<210>    104
<211>    238
<212>    PRT
<213>    Artificial Sequence

<220>
<223>    Human IgG light chain

<400>    104
Met Asp Met Arg Val Pro Ala Gln Leu Leu Gly Leu Leu Leu Leu Trp
1               5                   10                  15

Leu Ser Gly Ala Arg Cys Asp Ile Gln Met Thr Gln Ser Pro Ser Ser
            20                  25                  30

Leu Ser Ala Ser Val Gly Asp Arg Val Thr Ile Thr Cys Arg Ala Ser
        35                  40                  45

Gln Asp Val Asn Thr Ala Val Ala Trp Tyr Gln Gln Lys Pro Gly Lys
    50                  55                  60

Ala Pro Lys Leu Leu Ile Tyr Ser Ala Ser Phe Leu Tyr Ser Gly Val
65                  70                  75                  80

Pro Ser Arg Phe Ser Gly Ser Arg Ser Gly Thr Asp Phe Thr Leu Thr
                85                  90                  95

Ile Ser Ser Leu Gln Pro Glu Asp Phe Ala Thr Tyr Tyr Cys Gln Gln
            100                 105                 110

His Tyr Thr Thr Pro Pro Thr Phe Gly Gln Gly Thr Lys Val Glu Ile
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys Leu Glu
225                 230                 235

<210>   105
<211>   30
<212>   DNA
<213>   Artificial Sequence

<220>
<223>   Human IgG light chain

<400>   105
cctggcaagg ccccaaggct gctgatctac                                        30

<210>   106
<211>   30
<212>   DNA
<213>   Artificial Sequence

<220>
<223>   Human IgG light chain

<400>    106
gtagatcagc agccttgggg ccttgccagg                                        30

<210>    107
<211>    30
<212>    DNA
<213>    Artificial Sequence

<220>
<223>    Human IgG light chain

<400>    107
acaaaggtgg agatcaggag gaccgtggcc                                        30

<210>    108
<211>    30
<212>    DNA
<213>    Artificial Sequence

<220>
<223>    Human IgG light chain

<400>    108
ggccacggtc ctcctgatct ccacctttgt                                        30

<210>    109
<211>    30
<212>    DNA
<213>    Artificial Sequence

<220>
<223>    Human IgG light chain

<400>    109

gccaaggtgc agtggagggt ggataacgcc                                        30

<210>    110

<211>    30

<212>    DNA

<213>    Artificial Sequence

<220>

<223>    Human IgG light chain

<400>    110

ggcgttatcc accctccact gcaccttggc                                        30